# EUROPEAN PATENT APPLICATION

(11) **EP 1 797 836 A1**
(43) Date of publication of application: **20.06.2007**
(21) Application number: 04732628.5
(22) Date of filing: 13.05.2004
(51) Int. Cl.: A61B 18/00

(54) **HANDPIECE FOR MEDICAL/SURGICAL TREATMENTS**

(71) Applicant: Arcusa Villacampa, Francisco Javier, E-08029 Barcelona (ES); Franco Lissot, Mailin Auxiliadora, 08029 Barcelona (ES)
(72) Inventor: Arcusa Villacampa, Francisco Javier, E-08029 Barcelona (ES); Franco Lissot, Mailin Auxiliadora, 08029 Barcelona (ES)
(74) Representative: Morgades y Manonelles, Juan Antonio
(86) International application number: PCT/ES2004/000214
(87) International publication number: WO 2005/110260

(57) **Abstract**

It comprises a tubular body adapted for allowing an optical fibre to be passed through the inside and which comprises a gripping area and an application area. It is provided with integrated smoke suction means intended for aspiration of surgical smoke resulting from application of laser light on the tissue to be treated comprising a reduced length tubular conduit formed in the body of the piece and adapted to be coupled to a vacuum system. It may be provided with a luer cone termination adapted for coupling spinal puncture needles.

By applying a low vacuum in this handpiece side effects in the patient caused by smoke and fumes resulting from laser are effectively avoided, said smoke being aspirated at the same point where it is generated.

## Description

The present invention relates to a handpiece for medical surgical treatments which novel features provide many advantages, as it will be detailed in the present specification.

Handpieces adapted according to different specialties for tissue treating are known comprising a substantially tubular body which is configured for allowing an optical fibre cable to be passed through the inside. The substantially tubular body of the handpiece comprises a gripping area, having a larger diameter, and an application area, having a smaller diameter. At the free end of said application area the application end of the optical fibre projects.

The use of optical fibre in minimally invasive surgical treatments has shown to be very effective since it allows obtaining a deep penetration into the tissue as well as an optimum coagulation.

However, application of this technique results in undesirable surgical smoke resulting from interaction of laser light on the tissue. Thus, for example, in a case of laser disquectomy, smoke and fumes resulting from the action of the laser may give rise to a pressure inside the spine which may cause prolonged pain in the patient.

The present invention provides a handpiece alternative to handpieces which for the same purpose are currently used with which it is possible to overcome the above problems concerning generation of surgical smoke in the laser surgery processes.

The handpiece of the invention is of the previously described type, i.e. it is adapted for surgical diode laser application by means of optical fibre. It essentially comprises a substantially tubular body adapted in such a way that an optical fibre may be passed through the inside. The tubular body has a gripping area and an application area and it comprises integrated smoke suction means intended to aspiration of surgical smoke resulting from application of laser light on the tissue being treated.

Preferably, said integrated smoke suction means comprise, according to the invention, a reduced length tubular conduit formed in the body of the piece adapted to be coupled to a vacuum system. This tubular conduit may be formed in the gripping area of the body of the piece and it may have a luer cone termination adapted for attaching spinal puncture needles. By applying low vacuum in this handpiece side effects in the patient caused by smoke and fumed resulting from laser are effectively avoided.

Furthermore, with the handpiece of the invention space saving and high effectiveness are achieved since smoke is aspirated at the same point where it is generated.

From the detailed description of a preferred embodiment of a handpiece for surgical diode laser application by means of optical fibre according to the present invention the features and the advantages thereof will be clear. This description will be given hereinafter by way of a non-limiting example, with reference to the drawings enclosed in the present specification.

In this drawings:
Fig. 1 is a longitudinal sectional view of one embodiment of a handpiece according to the invention;
Fig. 2 is an enlarged elevational view of an area where liquid is fed into to the piece; and
Fig. 3 is a side elevational view of one embodiment of a handpiece according to the invention with luer cone termination and in which a spinal puncture needle has been provided.

Reference numerals used herein for describing the preferred embodiment of the handpiece of the present invention are listed below:
(1) handpiece;
(2) optical fibre;
(3) gripping area;
(4) application area;
(5) first end of the gripping area;
(6) threaded plug;
(7) second end of the gripping area;
(8) mouthpiece;
(9) cannula;
(10) smoke suction means;
(11) reduced length tubular conduit;
(11a) first length of the tubular conduit;
(11b) second length of the tubular conduit;
(12) inner hole of the gripping area;
(13) enlarged portion;
(14) vacuum conduit; and
(15) spinal puncture needle.

The example shown in the figures, and which it will be described below, refers to a handpiece which has been generally referenced by (1) in the figures. The handpiece (1) is designed for surgical diode laser application by means of highly flexible optical fibre with core diameters ranging from 50 µm to 1500 µm and it can receive both low numerical aperture fibres for good cutting and high numerical aperture fibres for coagulation and vaporization.

The handpiece (1) that is shown by way of a non-limiting example in the figures herein attached comprises a substantially tubular body adapted for allowing an optical fibre (2) to be passed through the inside thereof.

The substantially tubular body of the handpiece (1) has a gripping area (3) and an application area (4). The gripping area (3) is made of anodised aluminium and it has an ergonomic design so that operator can hold it in a comfortable way. Said gripping area (3) has a first end (5) through which the optical fibre (2) is inserted having a threaded plug (6). The threaded plug (6) is received inside the first end (5) of the gripping area (3) of the handpiece (1). When screwing up the plug (6) into said first end (5) of the piece (1) the optical fibre (2) becomes entrapped in such a way that it is prevented from being axially moved inside the piece (1). Fastening of the optical fibre (2) by screwing of the plug (6) is carried out once the optical fibre (2) has been passed through the whole body of the piece (1) and projects slightly out of the end of the application area (4).

The gripping area (3) of the handpiece (1) has a second end (7) where a mouthpiece (8) is attached. The mouthpiece (8) has the purpose of receiving a cannula (9) that is the one defining said application area (4) of the handpiece (1). The cannula (9) may have any appropriate configuration adapted to allow an optical fibre (2) to be passed through the inside thereof.

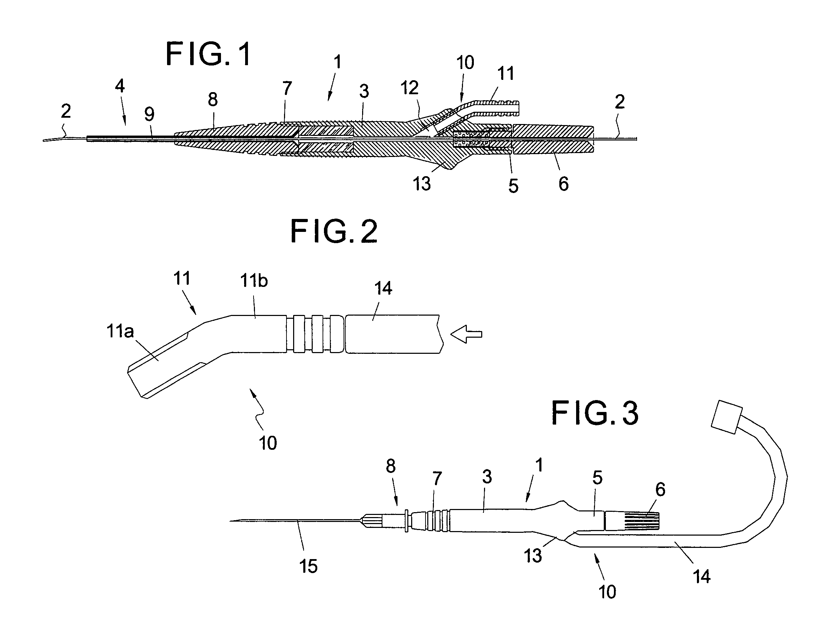

Integrated smoke suction means (10) are also provided. These smoke suction means (10) are adapted for aspirating surgical smoke caused by laser light application on the tissue that is being treated. For this purpose, said means (10) comprise a reduced length tubular conduit (11) formed in the body of the handpiece (1) which is suitably adapted to be coupled to a vacuum system through a conduit (14), as shown in detail in Fig. 2.

The tubular conduit (11) has a first length (11a) that is threadingly housed into an inner hole (12) of the gripping area (3) of the handpiece (1). Said inner hole (12) is formed in an enlarged portion (13) of said gripping area (3) in the handpiece (1) and it forms an angle to the longitudinal axis of the piece (1). Said first length (11a) of the conduit (11), formed in said gripping area (3) of the body of the piece (1), as noted above, extends to a second length (11b) forming an angle to the first length (11a), so that said second length (11b) of the conduit (11) is substantially parallel to the longitudinal axis of the piece (1).

One embodiment of a handpiece (1) has been depicted in Fig. n° 3 of the drawings in which the mouthpiece (8) has a luer cone termination for attaching a spinal puncture needle (15).

The configuration of the handpiece (1) shown in Fig. 1 provides a highly effective instrument in surgery such as laser treatment of disk herniation, avoiding undesirable painful side effects on the patient.

Once having been sufficiently described what the handpiece (1) for surgical diode laser application by means of optical fibre of the present invention consists in correspondence with the enclosed drawings, it will be understood that any detail modification considered as convenient may be introduced provided that the essential features of the invention summarized in the following claims are not altered.

## Claims

1. - Handpiece for medical surgical treatments (1) for diode laser application by means of optical fibre comprising a substantially tubular body adapted for allowing an optical fibre (2) may be passed through the inside, said substantially tubular body having a gripping area (3) and an application area (4), **characterized in that** it further comprises integrated smoke suction means (10) intended for aspiration of surgical smoke resulting from application of laser light on the tissue to be treated.

2. - Handpiece (1) as claimed in claim 1,
**characterized in that** said integrated smoke suction means (10) comprises a reduced length tubular conduit (11) formed in the body of the piece (1) and adapted to be coupled to a vacuum system.

3. - Handpiece (1) as claimed in claim 1 or claim 2, **characterized in that** said tubular conduit (11) is formed in said gripping area (3) of the body of the piece (1) .

4. - Handpiece (1) as claimed in claim 1, **characterized in that** it has a luer cone termination adapted for coupling spinal puncture needles (15).
